# EUROPÄISCHE PATENTSCHRIFT

(11) **EP 1 776 918 B1**
(45) Veröffentlichungstag und Bekanntmachung des Hinweises auf die Patenterteilung: **20.01.2010**
(21) Anmeldenummer: 06021581.1
(22) Anmeldetag: 14.10.2006
(51) Int. Cl.: A61B 1/00

(54) **Endoskop und Verfahren zu seiner Herstellung**
Endoscope and method for its manufacture
Endoscope et procédé de sa fabrication

(30) Priorität: 18.10.2005 DE 102005051207
(43) Veröffentlichungstag der Anmeldung: 25.04.2007
(73) Patentinhaber: Karl Storz GmbH & Co. KG, 78532 Tuttlingen (DE)
(72) Erfinder: Dahmen, Jan, 78606 Seitingen-Oberflacht (DE); Fürst, Frank, 78532 Tuttlingen (DE)
(74) Vertreter: Weller, Wolfgang

(56) Entgegenhaltungen:
- EP-A1- 0 025 969
- DE-A1-102004 008 458

## Beschreibung

Die Erfindung betrifft ein Endoskop, mit einem Endoskopschaft, dessen proximales Ende in einem Endoskopkopf aufgenommen ist, der einen Lichtleiteranschluss aufweist, wobei Lichtleiter vom Lichtleiteranschluss zwischen einem ersten Rohr und einem darin angeordneten zweiten Rohr durch einen Kanal des Endoskopschaftes zu dessen distalen Ende geführt sind und die Lichtleiter im Endoskopkopf in einem Hohlraum vom Endoskopschaft zum Lichtleiteranschluss geführt sind, wobei der Kanal und der Hohlraum mit einem Klebstoff gefüllt sind.

Ein derartiges Endoskop ist aus der EP 0 136 365 A1 bekannt.

Endoskope der eingangs genannten Art sind allgemein bekannt.

Diese Endoskope finden zum Beispiel Verwendung in der Arthroskopie, in der Bauch- und Brustkorbspiegelung, bei Leistenbrüchen sowie bei Gelenk- und Wirbelsäulenoperationen.

Endoskope der genannten Art weisen häufig zwei Rohre auf, ein äußeres Rohr und ein durchmessergeringeres inneres Rohr. Diese sind ineinander geschoben und bilden einen länglichen Endoskopschaft.

In einem Rohr ist die so genannte Optik untergebracht, meist ein Stablinsensystem oder ein elektronisches Bilderfassungs- und -umwandlungssystem.

Um das Operationsgebiet mit Licht auszuleuchten, ist im anderen Rohr bzw. im Raum zwischen den Rohren ein Lichtleitsystem angeordnet, meist ein Bündel an lichtleitenden Fasern. In diesem Kanal werden die Lichtleiter üblicherweise lose geführt.

Am proximalen Ende des Endoskopes sind die Lichtleiter in einem Lichtleiteranschluss im Endoskopkopf gefasst. Dieser Lichtleiteranschluss steht quer zur Schaftachse vom Endoskopkopf ab. Licht, das von der angeschlossenen Lichtquelle durch die Lichtleiter geschickt wird, tritt am distalen Ende des Endoskopes aus. Hierdurch wird das Operationsgebiet ausgeleuchtet.

Im Rahmen der minimal-invasiven Chirurgie, die durch kleinste Inzisionen am Körper erfolgt, ist es vorteilhaft, dass Endoskope in ihrem Aufbau möglichst schlank sind.

Daraus resultiert eine relativ geringe mechanische Stabilität des Schaftes, insbesondere gegen ein Biegen des Schafts.

Bei der Handhabung des Endoskopes, sei es während eines Eingriffes, sei es bei der nachfolgenden Reinigung und Autoklavierung, kann es durch Krafteinwirkung zu einer Biegung des Schaftes und somit auch des Optikrohres kommen, was zum Bruch bzw. Ausplatzen z. B. an den Stablinsen des Optiksystems führen kann.

Durch Bruchrisse an distalseitigen und proximalseitigen Dichtungsstellen des Faserkanals kann Feuchtigkeit in den Faserkanal eindringen, was zur Zerstörung der Fasern beim Autoklavieren führt. Die losen Fasern können bei Biegung des Schaftes brechen.

Die genannten Nachteile führen somit zu einer verkürzten Lebensdauer eines Endoskopes.

Bei der eingangs genannten EP 0 136 365 A1 wird in den Lichtleiteranschluss ein Klebemittel in Form eines optischen Kittes in flüssigem Zustand unter Druck eingeführt. Nachdem er sämtliche Hohlräume zwischen dem Lichtleiteranschluss und dem distalen Ende des Schaftes ausgefüllt hat, tritt der Kitt am distalen Ende aus. Auf diese Weise ist es möglich, die Stabilität des endoskopischen Sehrohres wesentlich zu erhöhen.

Es ist daher Aufgabe der vorliegenden Erfindung ein Endoskop bereitzustellen, das mit Klebstoff befüllt werden kann ohne dass die genannten Nachteile auftreten,

Erfindungsgemäß wird die Aufgabe dadurch gelöst, dass im Lichtleiteranschluss eine Fassung eingesetzt ist, die eine Mündung des Lichtleiteranschlusses aufweist, wobei im Bereich der Mündung eine als Bohrung ausgebildete Öffnung zum Einbringen des Klebstoffes ausgebildet ist.

Der Klebstoff kann nunmehr über eine separate Bohrung abseits der Mündung des Lichtleiteranschlusses, also der Stelle, an der die Lichtleiter enden, in dem Lichtleiteranschluss eingebracht werden.

Die Erfindung wird nachfolgend anhand eines ausgewählten Ausführungsbeispiels im Zusammenhang mit den beiliegenden Zeichnungen näher beschrieben und erläutert. Es zeigen:
- Figur 1: eine perspektivische Ansicht eines erfindungsgemäßen Endoskopes;
- Figur 2: einen teilweisen Längsschnitt durch das Endoskop von Figur 1, jedoch ohne Okular;
- Figur 3: das Endoskop der Figur 2 während eines erfindungsgemäßen Verfahrensschrittes; und
- Figur 4: einen Schnitt entlang der Linie IV-IV von Figur 3.

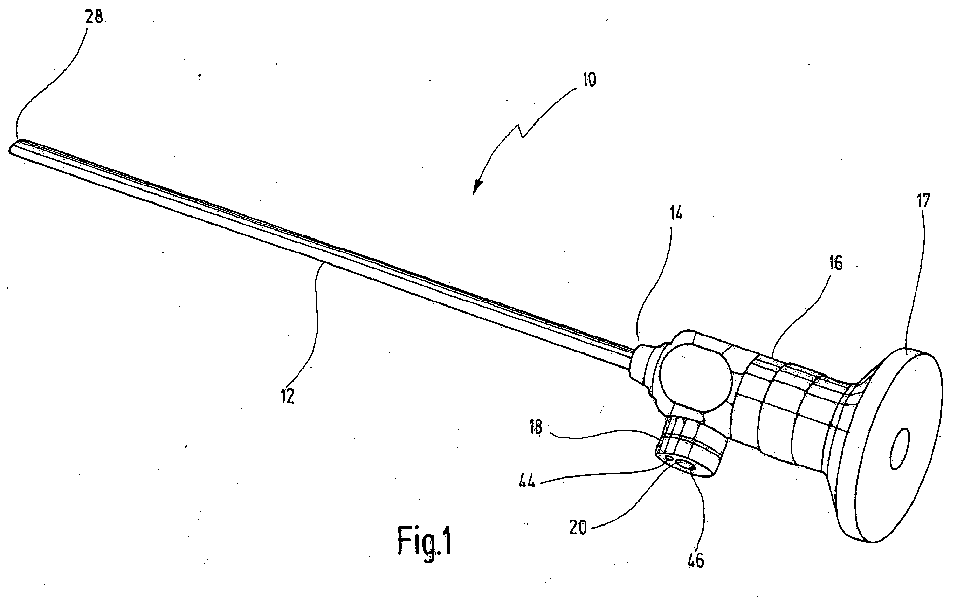

In Figur 1 ist ein Endoskop dargestellt, das in seiner Gesamtheit mit der Bezugsziffer 10 bezeichnet ist.

Das Endoskop 10 weist einen Endoskopschaft 12 auf. Dieser Endoskopschaft 12 ist mit seinem proximalen Ende 14 in einem Endoskopkopf 16 aufgenommen. Der Endoskopkopf 16 weist ferner einen seitlich abstehenden Lichtleiteranschluss 18 auf. Der Endoskopfkopf 16 trägt proximalseitig ein Okular 17. Der Lichtleiteranschluss 18 dient einer Aufnahme eines proximalen Endes von Lichtleitern 20 in Form eines Bündels oder Stranges von lichtleitenden Glasfasern.

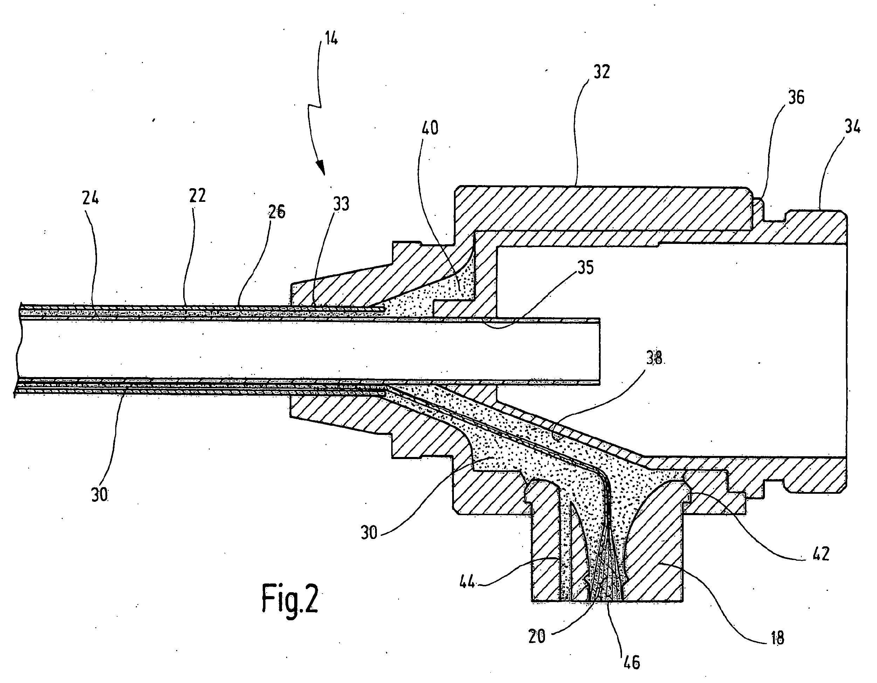

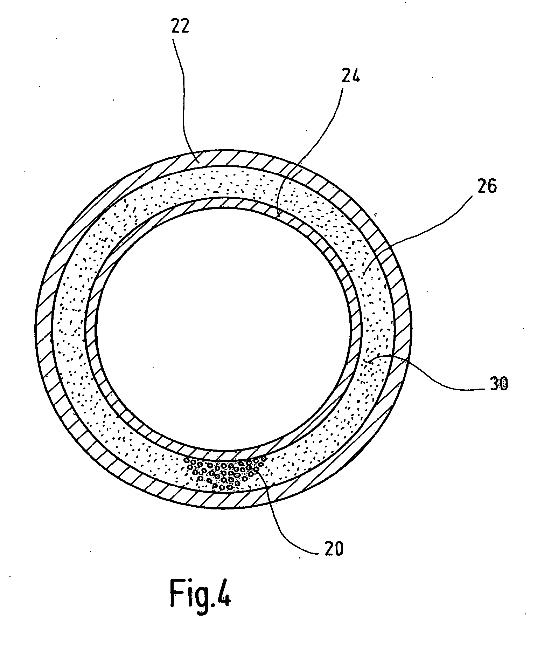

In den Figuren 2 bis 4 ist ersichtlich, dass der Endoskopschaft 12 mehrteilig aufgebaut ist.

Der Endoskopschaft 12 weist ein erstes äußeres Rohr 22 auf. In dieses erste Rohr 22 ist ein durchmessergeringeres zweites Rohr 24 eingeschoben. Das erste Rohr 22 und das zweite Rohr 24 sind koaxial zueinander angeordnet und somit radial voneinander beabstandet, so dass sich im Endoskopschaft 12 ein ringförmiger Kanal 26 bildet. Die Lichtleiter 20 sind vom Lichtleiteranschluss 18 durch diesen Kanal 26 des Endoskopschaftes 12 zu seinem distalen Ende 28 geführt. In dem erfindungsgemäßen Endoskop ist dieser Kanal 26 zusätzlich mit einem Klebstoff 30 gefüllt.

Der Endoskopkopf 16 ist, wie in Figur 2 dargestellt, ebenfalls mehrteilig aufgebaut. Der Endoskopkopf 16 weist eine erste Hülse 32 und eine zweite Hülse 34 auf. Die erste Hülse 32 weist den Lichtleiteranschluss 18 auf, der quer von dem Endoskopkopf 16 absteht.

Die erste Hülse 32 verjüngt sich zum Schaft 12 hin. Dabei ist das erste Rohr 22 so in der Verjüngung 33 aufgenommen, dass es etwas in die erste Hülse 32 hineinragt.

Die zweite Hülse 34 weist einen Ringflansch 36 auf, an dem die erste Hülse 32 proximal dicht anliegt.

Das zweite Rohr 24 erstreckt sich weiter in den Endoskopkopf 16 hinein als das erste Rohr 22. Proximal ist das zweite Rohr 24 in einer Öffnung 35 in der zweiten Hülse 34 dicht aufgenommen. Dabei erstreckt sich das zweite Rohr 24 in die zweite Hülse 34 hinein. Die zweite Hülse 34 weist darüber hinaus eine Schräge 38 auf. Diese Schräge 38 verjüngt sich distalseitig gesehen so, dass zwischen der ersten Hülse 32 und der zweiten Hülse 34 ein Hohlraum 40 gebildet ist.

In dem Lichtleiteranschluss 18 ist, wie aus Figur 2 ersichtlich, eine Fassung 42 eingesetzt. Von diesem Lichtleiteranschluss 18 sind die Lichtleiter 20 durch den Hohlraum 40 geführt. Von dort aus sind die Lichtleiter 20 durch den Kanal 26 zum distalen Ende 28 des Endoskopschaftes 12 geführt. Sowohl der Kanal 26 als auch der Hohlraum 40 sind in dem erfindungsgemäßen Endoskop 10 mit dem Klebstoff 30 gefüllt.

Die Fassung 42 weist ferner eine Öffnung 44 auf, die einem Einbringen des Klebstoffes 30 dient. Dabei ist diese Öffnung 44 als eine Bohrung im Bereich einer Mündung 46 des Lichtleiteranschlusses 18 ausgebildet, die eine Verbindung zwischen dem Hohlraum 40 und der Außenseite schafft.

Nachfolgend wird nun ein Verfahren zum Herstellen des Endoskopes 10 näher beschrieben.

In einem ersten Schritt werden das erste Rohr 22, das zweite Rohr 24, der Lichtleiteranschluss 18, die Lichtleiter 20, der Klebstoff 30, die erste Hülse 32 und die zweite Hülse 34 bereitgestellt.

Die Lichtleiter 20 werden in das Innere des ersten Rohres 22 geführt, das mit der ersten Hülse 32 fest verbunden ist. Die Lichtleiter 20 stehen dabei noch seitlich über den Lichtleiteranschluss 18 vor. Dann wird von proximal der Zusammenbau aus zweitem Rohr 24 und Hülse 34 in das Rohr 22 eingeführt, bis die zweite Hülse 34 in dem Ringflansch 36 zu liegen kommt. Auf diese Weise werden der Endoskopschaft 12 und der Endoskopkopf 16 zusammengebaut. Das erste Rohr 22 ist dabei von dem zweiten Rohr 24 so beabstandet, dass sich zwischen diesen beiden der Kanal 26 bildet.

Im Anschluss hieran wird im zweiten Rohr 24 eine Optik in Form von Linsen oder Stablinsen angeordnet.

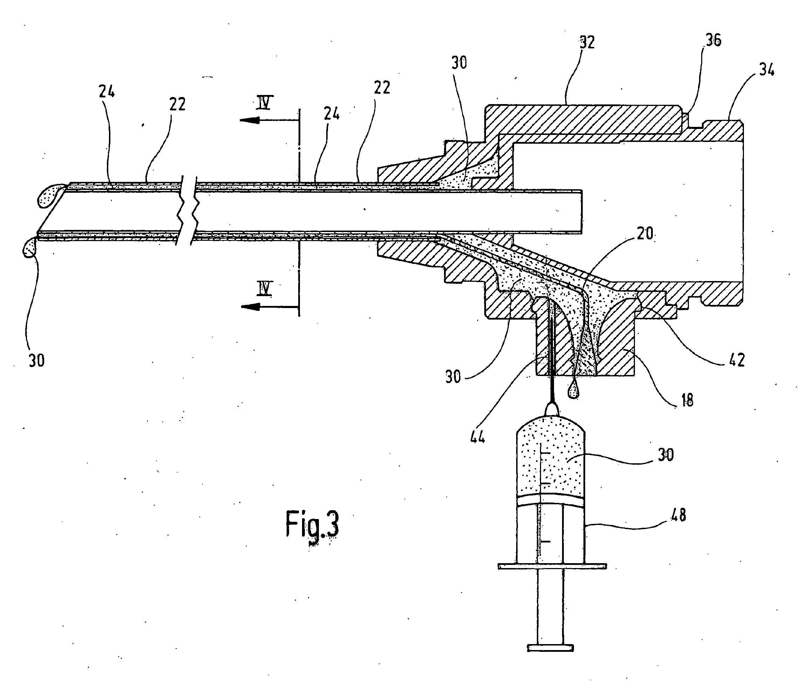

In Figur 3 ist dargestellt, dass der Lichtleiteranschluss 18 die Öffnung 44 im Bereich seiner Mündung 46 aufweist. Hierdurch lässt sich vorteilhaft zunächst der Hohlraum 40 des Endoskopkopfes 16 mit dem Klebstoff 30 füllen. In Figur 3 geschieht dies mittels einer Spritze 48. Dabei wird Luft aus dem Endoskop 10 zum distalen Ende 28 hin verdrängt. Bei einem entsprechend gleichmäßigen Füllen des Endoskopes 10 mit dem Klebstoff 30 werden die im Hohlraum 40 und im Kanal 26 geführten Lichtleiter 20 gleichmäßig mit dem Klebstoff 30 überzogen. Es wird so lange Klebstoff 20 eingefüllt, bis dieser am distalen Ende austritt.

Der Klebstoff 30 wird nun ausgehärtet. Dies geschieht bei einem Einkomponenten-Klebstoff beispielsweise durch eine entsprechende Temperaturerhöhung oder bei einem Mehrkomponenten-Klebstoff im Verlauf von Polymerisationsreaktionen.

In einem letzten Verfahrensschritt wird das Endoskop 10 nach Aushärten des Klebstoffes 30 an seinem distalen Ende 28 und an der Mündung 46 des Lichtleiteranschlusses 18 abgeschliffen und poliert.

## Patentansprüche

1. Endoskop, mit einem Endoskopschaft (12), dessen proximales Ende (14) in einem Endoskopkopf (16) aufgenommen ist, der einen Lichtleiteranschluss (18) aufweist, wobei Lichtleiter (20) vom Lichtleiteranschluss (18) zwischen einem ersten Rohr (22) und einem darin angeordneten zweiten Rohr (24) durch einen Kanal (26) des Endoskopschaftes (12) zu dessen distalen Ende (28) geführt sind und die Lichtleiter (20) im Endoskopkopf (16) in einem Hohlraum (40) vom Endoskopschaft (12) zum Lichtteiteranschluss (18) geführt sind, wobei der Kanal (26) und der Hohlraum (40) mit einem Klebstoff (30) gefüllt sind, **dadurch gekennzeichnet, dass** im Lichtleiteranschluss (18) eine Fassung (42) eingesetzt ist, die eine Mündung (46) des Lichtleiteranschlusses (18) aufweist, wobei im Bereich der Mündung (46) eine als Bohrung ausgebildete Öffnung (44) zum Einbringen des Klebstoffes (30) ausgebildet ist.

## Claims

1. Endoscope having an endoscope shaft (12) whose proximal end (14) is held in an endoscope head (16) which has an optical waveguide connection (18), wherein optical waveguides (20) being passed through a channel (26) in the endoscope shaft to its distal end (28) from the optical waveguide connection (18) between a first tube (22) and a second tube (24) which is arranged in it, and wherein the optical waveguides (20) are passed from the endoscope shaft (12) to the optical waveguide connection (18) in a cavity (40), wherein said channel (26) and said cavity (40) are filled with an adhesive (30), **characterized in that** a fitting (42) is inserted into the waveguide connection (18) comprising a mouth (46) of the waveguide connection (18), wherein an opening (44) designed as a bore is provided in the area of the mouth (46) for introducing the adhesive (30).

## Revendications

1. Endoscope, avec un corps d'endoscope (12) dont l'extrémité proximale (14) est logée dans une tête d'endoscope (16) qui présente un raccord de fibres optiques (18), des fibres optiques (20) étant guidées du raccord de fibres optiques (18) à travers un canal (26) du corps d'endoscope (12) vers son extrémité distale (28) entre un premier tube (22) et un deuxième tube (24) disposé dans celui-ci et les fibres optiques (20) étant guidées dans la tête d'endoscope (16) du corps d'endoscope (12) vers le raccord de fibres optiques (18) dans une cavité (40), le canal (26) et la cavité (40) étant remplis avec un produit adhésif (30), **caractérisé en ce que** dans le raccord de fibres optiques (18) est insérée une douille (42) qui présente une embouchure (46) du raccord de fibres optiques (18), une ouverture (44) réalisée sous la forme d'un perçage étant formée dans la zone de l'embouchure (46) pour l'introduction du produit adhésif (30).
